# EUROPEAN PATENT APPLICATION

(11) **EP 1 900 387 A1**
(43) Date of publication of application: **19.03.2008**
(21) Application number: 07116619.3
(22) Date of filing: 18.09.2007
(51) Int. Cl.: A61M 16/00

(54) **Breathing gas delivery system with user feedback**

(30) Priority: 18.09.2006 US 845392 P; 27.02.2007 US 891772 P
(71) Applicant: INVACARE CORPORATION, Elyria, OH 44036-2125 (US)
(72) Inventor: Messenger, Robert W., Lakewood, OH 44107 (US); Hudgel, David W., Harrison Township, MI 48045 (US); Golish, Joseph A., Pepper Pike, OH 44124 (US); Felty, David G., Parma, OH 44134 (US)
(74) Representative: Ganguillet, Cyril

(57) **Abstract**

A method for providing feedback to a user of a breathing gas delivery system includes collecting usage data during use of the breathing gas delivery system, comparing the usage data to a therapeutic target, generating feedback based on the comparison of the usage data to the therapeutic target; and communicating the feedback to the user. An apparatus (10) for providing feedback includes a user interface (18) in communication with the air flow source (14) and configured to be coupled to a user to provide air from the air flow source to the user, a controller (12) having memory for storing a therapeutic target, one or more usage data inputs. The controller is configured to compare usage data to the therapeutic target to determine feedback for the user. A feedback communicator presents the feedback to the user.

## Description

### Cross Reference to Related Applications

This application claims the benefit of priority from U.S. Provisional Patent Application Serial Nos. 60/845,392, filed on September 18, 2006 and 60/891,772, filed on February 27, 2007, both of which are incorporated herein by reference in their entirety for all purposes.

### Background

Obstructive sleep apnea is an airway breathing disorder caused by relaxation of the muscles of the upper airway to the point where the upper airway collapses or becomes obstructed by the soft tissue supported by these same muscles. It is known that obstructive sleep apnea can be treated through the application of pressurized air to the nasal passages of a user. The application of pressurized air forms a pneumatic splint in the upper airway of the user thereby preventing the collapse or obstruction thereof. Devices that provide the pressurized breathing gas are known as positive airway pressure, or PAP, devices.

### Summary

A method for providing feedback to a user of a breathing gas delivery system includes collecting usage data during use of the breathing gas delivery system, comparing the usage data to a therapeutic target, generating feedback based on the comparison of the usage data to the therapeutic target; and communicating the feedback to the user. An apparatus for providing feedback includes a user interface in communication with the air flow source and configured to be coupled to a user to provide air from the air flow source to the user, a controller having memory for storing a therapeutic target, one or more usage data inputs. The controller is configured to compare usage data to the therapeutic target to determine feedback for the user. A feedback communicator presents the feedback to the user.

### Brief Description of the Drawings

In the accompanying drawings which are incorporated in and constitute a part of the specification, embodiments of the invention are illustrated, which, together with a general description of the invention given above, and the detailed description given below, serve to example the principles of this invention.

Figure 1 a functional block diagram illustrating a breathing gas delivery system that provides user feedback.

Figure 2 is a functional block diagram illustrating a breathing gas delivery system that provides user feedback.

Figure 3 a functional block diagram illustrating a breathing gas delivery system that provides user feedback.

Figure 4 is a flowchart illustrating one example procedure for operation of the breathing gas delivery system.

Figure 5 is a perspective view of a CPAP device that is capable of providing user feedback according to an embodiment of the present invention.

Figure 5A is close up view of a display of the CPAP device of Figure 5.

### Detailed Description

Prior to discussing the various embodiments, a review of the definitions of some exemplary terms used throughout the disclosure is appropriate. Both singular and plural forms of all terms fall within each meaning:

Pressurized Breathing Gas Respiratory Therapy Device, as used herein, includes, but is not limited to, all Positive Air Pressure (PAP) devices including Continuous PAP (CPAP), auto-adjust PAP, and bi-level devices, Proportional Positive Air Pressure (PPAP) devices, a ventilator device, a gas therapy device, an oxygen therapy device, or any device that is used to provide one or more than one pressure with any form of pressure variability to treat either obstructive or central apnea, or both (mixed or complex apnea).

"Logic," as used herein, includes but is not limited to hardware, firmware, software and/or combinations of each to perform a function(s) or an action(s), and/or to cause a function or action from another component. For example, based on a desired application or needs, logic may include a software controlled microprocessor, discrete logic such as an application specific integrated circuit (ASIC), or other programmed logic device. Logic may also be fully embodied as software.

"Software," as used herein, includes but is not limited to one or more computer readable and/or executable instructions that cause a computer or other electronic device to perform functions, actions, and/or behave in a desire manner. The instructions may be embodied in various forms such as routines, algorithms, modules or programs including separate applications or code from dynamically linked libraries. Software may also be implemented in various forms such as a stand-alone program, a function call, a servlet, an applet, instructions stored in a memory, part of an operating system or other type of executable instructions. It will be appreciated by one of ordinary skill in the art that the form of software is dependent on, for example, requirements of a desired application, the environment it runs on, and/or the desires of a designer/programmer or the like.

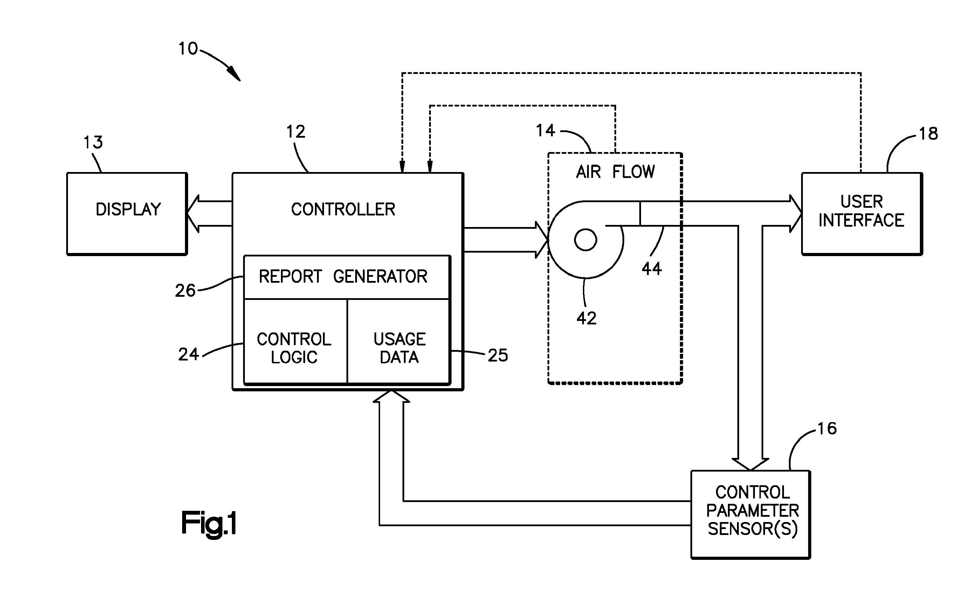

The systems and methods described herein are particularly suited for assisting the respiration of spontaneously breathing users, though they may also be applied to other respiratory regimens including, for example, central sleep apnea, mixed (complex) apnea, and acute and homecare ventilation. Referring now to Figure 1, a functional block diagram illustrating one embodiment of a breathing gas delivery system 10 is shown. The system 10 has a controller 12, a display 13, an air flow source 14, one or more control parameter sensors 16, and a user interface 18 through which the breathing gas is supplied to the user. The controller 12 includes control logic 24, storage means for storing usage data 25, and is configured to that manipulate the usage data to generate feedback for communication to the user via the a feedback communicator such as a display 13. The generation of feedback is performed in the described embodiment using a report generator 26. The air flow source 14 includes a blower 42 that provides a variable flow of air through an outlet 44 that is in fluid communication with the user interface. Breathable gas flows from the blower outlet 44 to a user interface 18. The user interface 18 can be any nasal mask, face mask, cannula, or similar device. The controller 12 is preferably processor-based and can include various input/output circuitry including analog-to-digital (A/D) inputs and digital-to-analog (D/A) outputs. According to the control logic 24, that may be implemented in software, the controller 12 controls the air flow source 14 based on the value of the control parameter sensed by the sensor(s) 16. The controller controls the air flow source by monitoring data from the control parameter sensors. The controller may also control the air flow source based on data obtained from the user interface 16 and operating parameters of the air flow source itself.

Usage data 25 is collected during operation of the system. The usage data may include a subset of data that is used in open or closed loop control of the air flow source as well as data that is collected for the purpose of providing usage feedback. The report generator 26 stores one or more therapeutic targets for a given user and compares the usage data with the therapeutic target to provide qualitative feedback to a user. For example, if a user has been given a target usage time of six hours per night, the report generator will compare the actual usage time with the target of six hours and provide feedback that communicates the results of the comparison. In this manner the feedback is qualitative because it provides more than just the raw usage time data, and informs a user how therapy is progressing compared to their specific therapeutic target.

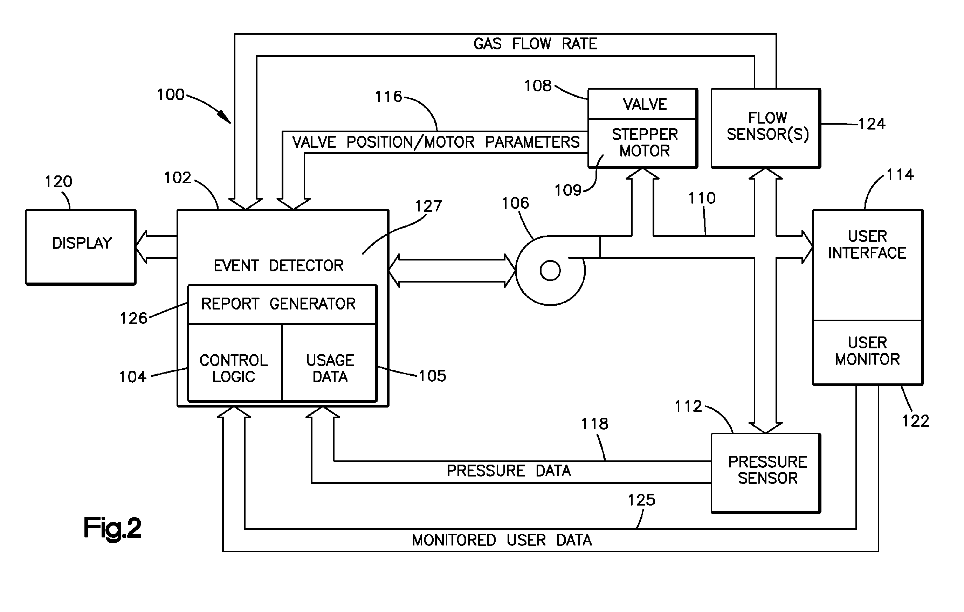

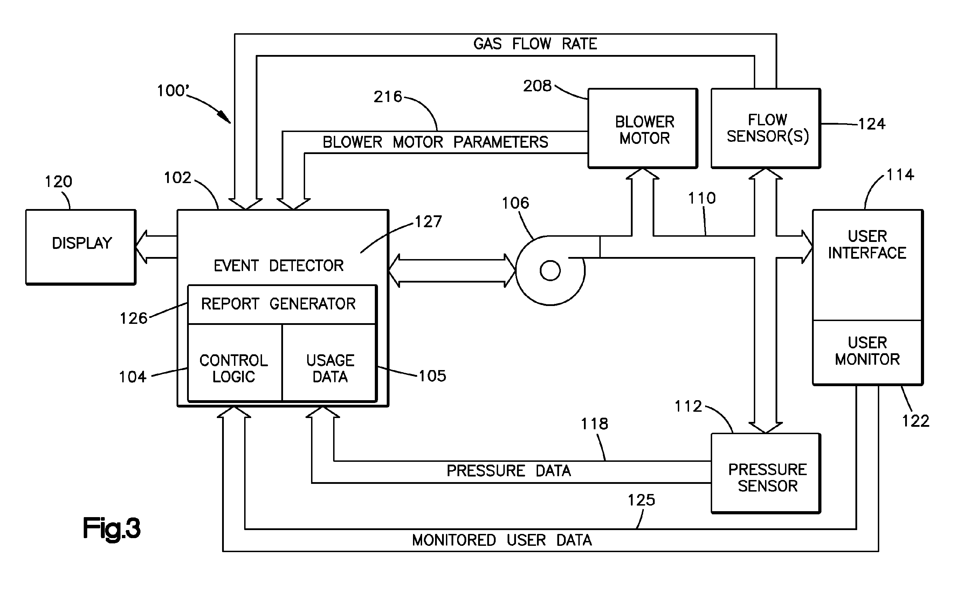

Figures 2 and 3 are functional block diagrams that include more detail about various components of a system for providing a breathing gas 100 (Figure 2) and 100' (Figure 3). The one or more control parameter sensors 16 (Figure 1) can include a variety of sensors, some of which are shown in Figures 2 and 3. Any or all of the sensors shown in Figures 2 and 3 may be included in the system and additional sensors may also be appropriately used in practice of the invention. For example, a pressure sensor 112 senses the pressure in the flow path between a blower 106 and the user interface 114. This pressure is associated with and indicative of the pressure in the user interface 114. A flow sensor 124 senses a flow rate of breathing gas through the flow path. Data from either or both of the pressure sensor 112 and flow sensor 124 can be used to deduce usage data, such as a level of obstruction present in the user's respiratory tract or a quality of fit between the user interface 114 and the user.

One or more user monitors 122 may be employed to collect usage data by directly sensing various physical parameters of the user. For example, the user monitor may include a heart rate monitor and/or a pulse-oximeter. The user monitors may be in wireless communication with the controller 102 to improve user comfort.

The air flow source 14 (Figure 1) may be controlled using any number of techniques. For example, as shown schematically in Figure 2, the air flow source may include a variable position poppet valve 108 controlled by a bi-directional stepper motor 109. To vary the amount of air flow that flows through the outlet to the user, the stepper motor moves the poppet valve within the air flow path to route a portion of the air through the outlet and re-circulate the remainder of the air to the blower. The controller 102 controls the stepper motor 109 to position the poppet valve 108 according to the pressure sensed by pressure sensor 112 and/or the gas flow sensed by the gas flow sensor 124. In addition, data regarding the valve position and stepper motor parameters is monitored as part of closed loop control. An example of such a system is described in more detail in U.S. Patent No. 7,152,598, and U.S. Patent Application Serial No. 11/157,089, filed June 20, 2005, both of which are incorporated herein by reference in their entireties.

Alternatively, as shown schematically in Figure 3, the air flow can be varied by directly controlling a blower motor 208, such as, for example, by pulse width modulation of the blower motor's control signal. The controller 102 controls the blower motor 208 according to the pressure sensed by pressure sensor 112 and/or the gas flow sensed by the gas flow sensor 124. Data regarding the blower motor's operation is monitored by the controller 102 as part of closed loop control of the blower motor. A system that utilizes this type of air flow source is described in U.S. Patent No. 6,990,980, which is incorporated herein by reference in its entirety.

The controller 102 may include an event detector 127 that evaluates any or all of the inputs to the controller shown in Figures 2 and 3. The event detector includes logic that detects occurrences of apnea, hypopnea, abnormal breathing rates or cycles, or any number of respiratory events related to breathing gas therapy delivered by the system. The event detector logs occurrences of the various respiratory events and the information logged by the event detector is accessible to the report generator 126.

To provide feedback to the user, the report generator inputs usage data or deduces usage data from any or all of the sensors shown in Figures 2 and 3 as well as the event detector 127 if present. For example, usage data can be obtained or deduced from the user interface, user monitor, flow sensor, pressure sensor, sensed valve position, sensed stepper motor operating parameters, and sensed blower motor operating parameters. The report generator is capable of storing one or more therapeutic targets, such as a duration of use per night, a maximum number of abnormal breathing events, or any number of other targets. The report generator may also be capable of extrapolating information to be presented as feedback from usage data. For example, the report generator may deduce a usage time or mask fit quality from flow or pressure data. The report generator 126 compares usage data to the corresponding therapeutic target and presents the results of this comparison to the user on the display 120.

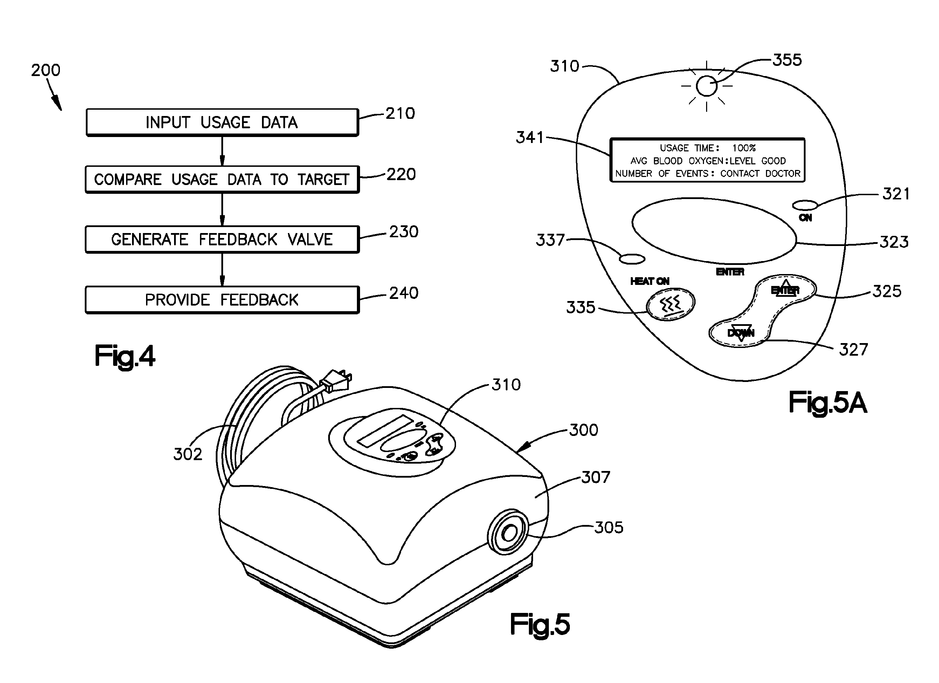

Referring now to Figure 4, the operation of the system for providing user feedback will be described with reference to the flowchart illustrated therein. In the flowchart, the rectangular elements denote processing blocks and represent software instructions or groups of instructions. The flow diagrams shown and described herein do not depict syntax of any particular programming language. Rather, the flow diagrams illustrate the functional information one skilled in the art may use to fabricate circuits or to generate software to perform the processing of the system. It should be noted that many routine program elements, such as initialization of loops and variables and the use of temporary variables are not shown.

Figure 4 is a flowchart illustrating a method 200 that provides feedback to a user of a system for providing a breathing gas. At 210, usage data is input to the report generator. As discussed above, the usage data can be obtained, for example, from monitors on the patient, sensors in the system, or control parameters of the air flow source. At 220 the usage data is compared to a therapeutic target for the user. The therapeutic target may be input by a therapist or doctor and may be based on baseline information gathered from the user prior to the start of therapy such as, for example, a typical number of respiratory events experienced per night or an average blood oxygen level during sleep. As discussed above, usage data may need to be combined or manipulated to be compared to the target. At 230, a feedback value that represents the results of the comparison between the usage data and the therapeutic target is generated. The feedback may be, for example, an indication as to whether a particular characteristic of usage falls within an acceptable range. The feedback value may be, for example, a scaled version of the usage data when compared to the therapeutic target. The feedback value may be, for example, a difference between usage data during a given period of use and baseline data collected from the patient prior to therapy. At 240, the feedback value is provided. In the described embodiments, the feedback value is displayed on an alphanumeric display or in the form of one or more colored lights. However, any method of communicating the feedback value to the user, including, for example, audible signals or removable memory media that can be accessed by a user's computer to provide the feedback can be used in practice of the present invention.

The display 120 may be a part of a CPAP unit 300 shown in Figures 5 and 5A. The CPAP unit includes a power source 302, a housing 307 that contains the blower, a gas outlet 305 through which the therapeutic gas exits the unit, and an operating interface panel 310. The operating interface panel includes a power button 321, heater button 335, increment/decrement button 325/327, and an "ENTER" button 323. The panel 310 includes an alphanumeric display 341 that can display messages that present feedback values and may also include an illuminated light 355 that shows the color red or green depending on whether the feedback falls within a range of acceptable values or outside the range of acceptable values.

The following examples are intended to further describe the breathing gas delivery system with feedback. The scope of embodiments that can be used to practice the breathing gas delivery system with feedback is not limited to the examples. In one embodiment the breathing gas delivery system usage time may be monitored. This usage time can be monitored, for example, by recording a start and stop time. The usage time data can be converted to feedback by comparing the usage time to a stored target usage time and calculating a relative usage time, such as 90% of the target usage time was achieved for a given night's usage. This relative usage time data can be recorded over a predetermined interval, such as weekly. A running average of relative usage for the interval and/or the relative usage for the previous night can be communicated to the user on a display or using another feedback communication mechanism as will be discussed below.

In an auto-adjusting PAP device, pressure and/or gas flow may be monitored over the course of one or more usage periods. Pressure and/or flow levels are recorded and used to generate feedback corresponding to an appropriate pressure setting for use with a PAP device that has a single pressure setting that is manually entered. This can allow a user to switch to a more inexpensive PAP device once a pressure level setting has been determined for them.

By monitoring pressure and/or gas flow and valve position, a combination of the pressure data and the valve position data can be used to generate feedback related to a leak condition. For example, with the mask properly installed, an initial baseline pressure and/or gas flow and valve position can be recorded over a short time duration while the user is awake and it can be verified that the mask is properly installed. In subsequent usages, the pressure and valve position when the user initially places the mask on his face can be compared to the baseline pressure/flow/position data. If the pressure/flow/position varies from the baseline data, the user may be alerted to adjust the mask or to check for a leak in the system.

Apnea events can be detected and recorded by monitoring valve position or gas flow. A lack of valve movement or a drop in gas flow over a period of time indicates that the user has stopped breathing. Valve movement and/or gas flow data can thus be converted to feedback corresponding to a total number of apnea events that occurs over a given period of time. A number of apnea events may also be presented as indexed over predetermined intervals, such as an average number of events per hour. Additional information such as the duration of apnea events or a comparison with data concerning apnea events experienced by the user in the past may also be communicated to the user. In addition, instructions to the user may be stored in memory that are correlated to a given quantity of apnea events. Instructions corresponding to a detected quantity of apnea events can be retrieved from memory and displayed to a user.

The position of the valve and/or gas flow rate may also be used to detect hypopnea, or under-breathing, events in which insufficient air is taken in during inspiration. The valve position or gas flow rate may indicate that inspiration is of insufficient duration or pressure. As with apnea events, a total number of hypopnea events and/or a comparison with data concerning apnea events experienced by the user in the past can be provided. A number of hypopnea events may also be presented as indexed over predetermined intervals, such as an average number of events per hour. In addition, instructions to the user may be stored in memory that are correlated to a given quantity of hypopnea events. Instructions corresponding to a detected quantity of hypopnea events can be retrieved from memory and displayed to a user.

Usage data can be in the form of user physical parameters, such as, for example, blood oxygen saturation level and heart rate can be monitored using a pulse-oximeter. Signals from the pulse-oximeter can be used to generate feedback relating to an overall quality of respiration as well as pinpointing stress events that occurred during usage. An average, high, or low oxygen or heart rate level can be determined as well as an indexed value that gives an average over a given interval of time. Any of these values as well as an indication of the overall quality or number of occurrences of stress events can be communicated to the user. In addition, instructions to the user may be stored in memory that are correlated to a given range of oxygen saturation level or heart rate. Instructions corresponding to a detected level can be retrieved from memory and displayed to a user.

Feedback indicator mechanisms can include visual displays, printouts, or transmission of the feedback indicator by another method. The pressurized breathing gas respiratory therapy device may include a display that displays an alphanumeric message. Alphanumeric messages may include, for example, an indication of relative time usage with respect to a given target, an appropriate pressure setting for the device, an indication as to whether a leak exists in the system, a number of apnea or hypopnea events, or an oxygen saturation level or heart rate. The alphanumeric display may also display stored instructions that correspond to actions that should be taken by the user based on the observed usage data. For example, the instructions may instruct the user to check that the mask is properly fitted to the face or to see a sleep specialist, or communicate an overall quality of sleep that has been attained based on any or all of the above outlined usage data. A numeric display may display a number that is correlated to a feedback indicator. A user may be given an index that correlates the display numbers with feedback messages. Icons may be displayed that symbolize the various feedback indicators. Colored lights, such as red, yellow, and green, or chimes or buzzers, can be activated to indicate whether usage data fell within an acceptable, marginally acceptable, or unacceptable range. A printout may be provided for the user to take to their sleep specialist. The feedback indicator may be transmitted to a separate storage device such as a memory card or to a communication device such as a PDA, cell phone, or computer.

While the present invention has been illustrated by the description of embodiments thereof, and while the embodiments have been described in considerable detail, it is not the intention of this specification to restrict or in any way limit the scope of the appended claims to such detail. Additional advantages and modifications will readily appear to those skilled in the art. Therefore, the invention, in its broader aspects, is not limited to the specific details, the representative apparatus, and illustrative examples shown and described. Accordingly, departures may be made from such details without departing from the spirit or scope of the applicant's general inventive concept.

## Claims

1. A method of providing feedback to a user of a breathing gas delivery system, the method comprising the steps of:
collecting usage data during use of the breathing gas delivery system;
comparing the usage data to a therapeutic target;
generating feedback based on the comparison of the usage data to the therapeutic target; and
communicating the feedback to the user.

2. The method of claim 1 comprising the step of manipulating the usage data and comparing the manipulated usage data to the therapeutic target.

3. The method of claim 2 wherein the step of manipulating the usage data is performed by comparing the usage data to predefined respiratory event criteria and determining that a respiratory event has occurred based on the comparison of the usage data to the event criteria.

4. The method of any one of claims 1 to 3 comprising the step of storing a therapeutic target for the user.

5. The method of claim 4 wherein the step of storing a therapeutic target for a user is performed by storing a target based on baseline data collected from the user.

6. The method of any one of claims 1 to 5 wherein the step of collecting usage data comprises recording a breathing gas delivery system activation and deactivation time.

7. The method of any one of claims 1 to 6 wherein the step of collecting usage data comprises recording a gas pressure in the breathing gas delivery system.

8. The method ofany one of claims 1 to 7 wherein the step of collecting usage data comprises recording a flow rate of gas through the breathing gas delivery system.

9. The method of any one of claims 1 to 8 wherein the step of collecting usage data comprises recording a physical parameter of a user.

10. The method of any one of claims 1 to 9 wherein the step of collecting usage data comprises recording an operating parameter of a breathing gas delivery system component.

11. The method of any one of claims 1 to 10 wherein the step of communicating the feedback to the user is performed by illuminating a light.

12. The method of any one of claims 1 to 10 wherein the step of communicating the feedback to the user is performed by displaying an alphanumeric message on the breathing gas deliver system.

13. A breathing gas delivery system comprising:
a user interface in communication with an air flow source and configured to be coupled to a user to provide air from the air flow source to the user;
a controller comprising:
memory for storing a therapeutic target;
one or more usage data inputs;
the controller configured to compare usage data to the therapeutic target to determine feedback for the user; and
a feedback communicator that presents the feedback determined by the controller to the user.

14. The breathing gas delivery system of claim 13 wherein the feedback communicator comprises an alphanumeric display.

15. The breathing gas delivery system of claim 13 or 14 wherein the feedback communicator comprises one or more lights.

16. The breathing gas delivery system of any one of claims 13 to 15 wherein at least one usage data input is in signal communication with a pressure sensor disposed in an air flow path along which the air provided to the user flows.

17. The breathing gas delivery system of any one of claims 13 to 16 wherein at least one usage data input is in signal communication with a flow sensor disposed in an air flow path along which the air provided to the user flows.

18. The breathing gas delivery system of any one of claims 13 to 17 comprising at least one user monitor that monitors a physical parameter of the user.

19. The breathing gas delivery system of any one of claims 13 to 18 comprising an event detector that logs an occurrence of breathing events based on usage data.
